# EUROPEAN PATENT SPECIFICATION

(11) **EP 3 443 848 B1**
(45) Date of publication and mention of the grant of the patent: **18.03.2020**
(21) Application number: 18168272.5
(22) Date of filing: 11.12.2014
(51) Int. Cl.: A23L 2/00, A23L 2/52, A61K 33/26

(54) **MINERAL WATER COMPOSITION CONTAINING BIOAVAILABLE IRON**
MINERALWASSERZUSAMMENSETZUNG ENTHALTEND BIOVERFÜGBAREM EISEN
COMPOSITION D'EAU MINÉRALE CONTENANT DU FER BIODISPONIBLE

(30) Priority: 11.12.2013 GB 201321923
(43) Date of publication of application: 20.02.2019
(62) Divisional of application: 14811880.5
(73) Proprietor: Snowdonia Research Sarl, 1840 Luxembourg (LU)
(72) Inventor: OLDKNOW, Chris, London, SW19 5LP (GB)
(74) Representative: Wilkinson, Stephen John

(56) References cited:
- EP-A2- 0 297 679
- WO-A1-2013/109516
- WO-A2-02/096225
- CN-A- 101 731 627
- DE-A1- 19 700 368
- US-A1- 2004 058 034

## Description

The present invention relates to a mineral water composition containing bioavailable iron. More particularly, it relates to an artificial or synthetic mineral water composition which provides oral, bioavailable iron that has use as a supplement to dietary iron.

Iron is an essential mineral nutrient for most forms of life. In the human body, iron is, *inter alia*, a component of haemoglobin which transports oxygen from the lungs to the tissues, a component of myoglobin which stores and diffuses oxygen in muscle cells, and a component of iron-sulphur proteins which are involved in the redox reactions of mitochondrial electron transport.

In the human diet, iron occurs in two forms: haem and non-haem. Haem iron, typically in the form of red meat animal products, is better absorbed by the body than non-haem iron. Sources of non-haem iron include cereals and vegetables.

Iron deficiency in a human arises when that human's requirements for iron are not met by the normal dietary intake. Men, typically, lose about 1 mg iron per day and this loss is normally replaced by dietary iron alone. Children have relatively high iron requirements because of the demand created by rapid growth. In women, childbirth and menstruation create significant demands for iron, with iron losses in women typically being about 2 mg per day. Such a loss, normally has to be replaced by dietary iron.

Many different oral iron-containing supplementation formulas are available, either as general food supplements or as specific iron treatments. Conventional oral treatments for anaemia use ferrous salts, for example ferrous sulphate, ferrous fumarate and ferrous gluconate. One problem with such ferrous iron formulations, which are typically provided in tablet form, is that they show variable bioavailability. Consequently, such ferrous formulations may not be absorbed readily by the body. In addition, ingestion of ferrous salts can cause, in some patients, a variety of symptoms, such as nausea, vomiting, diarrhoea, constipation and/or abdominal pain.

WO 2013/109516A discloses a beverage comprising supplemental iron. The composition disclosed comprises one or more iron (II) mineral sources, one or more edible high ferric ion reducing agents selected from the group consisting of ascorbic acid, edible ascorbic acid salts, edible ascorbic acid esters, erythorbic acid, edible erythorbic acid salts, and erythorbic acid esters to retard conversion of the iron (II) mineral sources from ferrous to ferric species, a citric acid and malic acid component to lower the pH of the composition to about 4.0 or less; and one or more calcium mineral sources which can be solubilized by the acids.

DE 19700368A discloses a beverage which comprises a divalent iron salt to give a Fe²⁺ ion concentration of 0.1 to 20 mg/l. The beverage is made acid to provide stability for the Fe²⁺ ion content.

The present invention is based on a discovery that certain mineral water compositions can be manufactured which have high levels of bioavailable iron.

The present invention provides a non-toxic artificial mineral water composition containing bioavailable iron, said composition consisting of an aqueous solution having a pH of from 1 to 5 containing ferrous ions at a concentration of from 100 to 300 mg/l and a non-toxic amount of Zn²⁺ ions wherein the concentration of Zn²⁺ ions is in the range of from 20 to 60 mg/l, and wherein the composition, when diluted to an iron concentration of 20 µm, has an iron uptake, measured as intracellular ferritin, of greater than 120 ng/mg protein, wherein iron uptake is determined by incubating human intestinal epithelial Caco-2 cells according to the method provided in the examples.

As stated above, the artificial mineral water composition contains ferrous ions at a concentration of 100 to 300 mg/l. Preferably, the ferrous ion concentration is at least 150 mg/l. According to a different embodiment, the ferrous ion concentration in the composition is not greater than 250 mg/l. According to a more preferred embodiment the ferrous ion concentration is in the range of 175 - 225 mg/l and, especially about 200 mg/l. A preferred source of ferrous ions is ferrous sulphate.

The mineral water composition of the invention may contain a non-toxic amount of at least one metal cation selected from Ca²⁺, Mg²⁺, and K⁺. If Ca²⁺ ions are present in the composition, they are preferably present in a concentration not greater than 200 mg/l. Preferably, the Ca²⁺ concentration is at least 100 mg/l and more preferably at least 150 mg/l. According to a different embodiment, the Ca²⁺ concentration does not exceed 190 mg/l, preferably not greater than 180 mg/l. Particularly preferred is a Ca²⁺ concentration of about 170 m/l. A preferred calcium source is CaSO₄2H₂O which may be dissolved in water or mineral solution acidified by the addition of sulphuric acid.

If Mg²⁺ ions are present in the composition they are typically present in a concentration which is not greater than 60 mg/l, preferably not greater than 50 mg/l. According to a different embodiment, Mg²⁺ ions are present in the composition at a concentration of at least 20 mg/l, preferably at least 30 mg/l and most preferably about 40 mg/l. A preferred magnesium source is magnesium sulphate.

According to an embodiment of the invention, the synthetic mineral water composition contains Ca²⁺ ions and Mg²⁺ ions. Typically, such a composition contains 100 to 200 mg/l Ca²⁺ ions and 20 to 60 mg/l Mg²⁺ ions, preferably 150 to 190 mg/l Ca²⁺ ions and 30 to 50 mg/l Mg²⁺ ions, more preferably about 170 mg/l Ca²⁺ ions and about 40 mg/l Mg²⁺ ions.

The synthetic mineral water composition of the present invention contains a non-toxic amount of Zn²⁺ ions, wherein the concentration of Zn²⁺ ions is in the range of from 20 to 60 mg/l. Preferably, the Zn²⁺ ion concentration does not exceed 50 mg/l. According to one preferred embodiment of the invention, the synthetic mineral water composition contains about 40 mg/l of Zn²⁺ ions. A preferred zinc source is zinc sulphate.

As stated above, the synthetic mineral water composition may contain a non-toxic amount of K⁺ ions. A typical source, of potassium is potassium sulphate. According to one preferred embodiment, potassium is provided in the form of the potassium salt of sorbic acid (hexa-2,4-dienoic acid) because of the preservative properties of potassium sorbate. Typically, potassium sorbate will be present in the synthetic mineral water composition at a concentration not exceeding 1800 mg/l and preferably not greater than 1600 mg/l. More preferably, potassium sorbate will be present in the composition at a concentration within the range of from 1000 to 1500 mg/l. According to one preferred embodiment of the invention, the synthetic mineral water composition has a potassium sorbate content of from 1300 to 1400 mg/l.

The synthetic mineral water composition of the invention may contain iron in the form of Fe³⁺ ions. We have found that if ferric (i.e. Fe³⁺) ions are present, they may be present in the composition at a concentration not greater than 70 mg/l and preferably not greater than 50 mg/l. Typically, the concentration of Fe³⁺ ions, if they are present in the composition, will be from 5 to 20% of the concentration of Fe³⁺ ions. The concentration of Fe³⁺ ions in the composition of the present invention may be about 10% of the concentration of Fe²⁺ ions. Thus, the artificial mineral water composition of the present invention may contain ferric ions at a concentration such that the ratio of the concentration of ferrous ions to the concentration of ferric ions in the solution is about 10:1. According to another embodiment, the artificial mineral water composition contains ferric ions and calcium ions such that the ratio of the concentration of ferrous ions to the concentrations of each of ferric ions and calcium ions (Fe²⁺:Fe³⁺:Ca²⁺) is about 10:1:8-9. According to a different embodiment, the artificial mineral water composition contains ferric ions and magnesium ions such that the ratio of the concentration of ferrous ions to the concentrations of each of ferric ions and magnesium ions (Fe²⁺:Fe³⁺:Mg²⁺) is about 10:1:2. According to a yet different embodiment, the synthetic mineral water composition of the invention contains ferric ions and zinc ions such that the ratio of the concentration of ferrous ions to the concentrations of each of ferric ions and zinc ions (Fe²⁺:Fe³⁺: Zn²⁺) is about 10:1:2.

The synthetic mineral water compositions of the present invention may contain one or more other metal cations in non-toxic amounts and which do not have a detrimental effect on iron uptake. Examples of such other metal cations include sodium and manganese. A typical source of sodium ions is sodium chloride. A typical source of manganese ions is manganese sulphate.

According to an embodiment, the composition of the invention contains one or more preservatives. A preferred preservative for use in the invention is potassium sorbate. However, one or more other preservatives may be used instead of, or in addition to, potassium sorbate.

The composition of the invention will also contain one or more suitable inorganic counterions. Such inorganic counterions, to be suitable, will be non-toxic at the concentrations used and will be soluble so as to maintain the essential and preferred metal ions discussed above in solution. The preferred inorganic counterion for use in the composition of the invention is sulphate anion although other anions, such as phosphate anion and/or chloride anion may be present in the mineral water composition.

The artificial mineral water composition has a pH value in the range of from 1 to 5, as stated above. Preferably, the pH of the composition will be in the range of from 2 to 5, more preferably from 2 to 4 and most preferably within the range of from 2.5 to 3.5. Typically, the pH of the composition will be adjusted by the addition of a suitable acid, for example sulphuric acid, malic acid, citric acid and mixtures thereof.

The artificial mineral water composition may be flavoured using additives known in the art. Preferred flavour additives include one or more fruit juice, such as apple juice or citrus juice, and/or Vitamin C. Typically, apple flavouring will be provided by the addition of apple concentrate, for instance a concentrate obtained by macerating and pressing apples and then dehydrating the juice obtained to concentrate it. Optionally, the juice expelled by the pressed apples may be treated to remove starch and pectin prior to concentration. Fruit juices and concentrates will contain naturally-occurring sugars. It is also possible to include one or more sugars and/or other sweetening agent to the mineral water composition.

The artificial mineral water composition of the present invention may be prepared by the addition to sterile water of an appropriate amount of a sterile concentrated stock solution of ferrous salt and appropriate amounts of one or more of sterile concentrated stock solutions of other desired salts and of other desired additives. Preferably, the water to which the stock solutions are added is acidified, prior to the addition, of an amount of sulphuric acid to lower the pH to a value of less than 4.0, preferably pH 3.5 or lower, to aid the stability of the prepared solution. This is especially useful if the desired artificial mineral water composition is to contain calcium ions. In view of the low solubility of calcium sulphate, it is not possible to prepare concentrated stock solutions of this salt. We prefer, therefore, to add solid calcium sulphate dihydrate to an acidified solution, as described above, and allow this to dissolve therein. The addition of the calcium sulphate dihydrate to the acidified solution may be carried out before, during or after the addition of the concentrated stock solutions of the other salts and optional additives. The pH of the obtained solution may, if necessary, be adjusted to the desired pH value by the addition of a further amount of sulphuric acid, or by the addition of malic acid and/or citric acid.

The artificial mineral water composition of the present invention contains bioavailable iron, i.e. iron in a form that is easily taken up by the body after ingestion of the composition. The composition, when diluted to an iron concentration of 20 µm, has an iron uptake, measured as intracellular ferritin, of greater than 120 ng/mg protein.

Iron absorption in the body is known to take place in the duodenum region of the small intestine. Iron uptake, according to the present invention, may be determined by an *in vitro* method which employs Caco-2 cells to simulate duodenal absorption. Caco-2 cells resemble the human intestinal epithelial cells that line the inner surfaces of the small intestine which absorb nutrients from ingested food. Caco-2 cells, which express most proteins for iron uptake and transport in humans, are widely used in the pharmaceutical industry for drug absorption studies. A method of predicting iron availability in a food sample which uses ferritin formation in Caco-2 cells as an indicator is disclosed in US 6,017,713. This patent discloses an *in vitro* digestion/cell culture model using Caco-2 cells.

According to the method used herein, which is described in more detail in the Examples, Caco-2 cells are incubated in the presence of the artificial mineral water composition after this has been subjected to simulated stomach digestion conditions and then the cells are harvested and lysed to liberate the ferritin produced. The amount of ferritin may then be assayed using, for instance, a ferritin enzyme-linked immunosorbent assay (ELISA) technique. Such techniques are well-known in the art and ferritin ELISA kits are commercially available. A typical technique may utilise rabbit anti-ferritin for the solid phase (microtitre wells) immobilisation and rabbit anti-ferritin in the antibody-enzyme (alkaline phosphatase) conjugate solution. The test sample is allowed to react simultaneously with the antibodies, resulting in ferritin molecules being sandwiched between the solid phase and the enzyme-linked antibodies. After a period of incubation, followed by washing, the immobilized ferritin-antibody complex is subjected to colour development which is measured spectrophotometrically. The concentration of ferritin in the sample tested is directly proportional to the measured colour intensity.

As stated above, the artificial mineral water composition of the present invention, when diluted to an iron concentration of 20 µM, has an iron uptake, measured as intracellular ferritin, which is greater than 120 ng/mg protein. Preferably, the iron uptake is greater than 150 ng/mg protein. We have, for instance, been able to prepare artificial mineral water compositions which demonstrate iron uptake values of above 190 ng/mg protein as shown herein in the Examples. It is known to persons skilled in the art that the higher the uptake of iron, the greater is the chance of increased absorption for utilisation in the body.

The artificial mineral water compositions of the invention will, after preparation, be stored in air-tight, prior-sterilised containers, such as bottles or plastic sachets.

The mineral water compositions of the invention have use as supplements to dietary iron intake. They may, also, be used in admixture with other dietary supplements.

### EXAMPLES

Various different aqueous iron solutions were prepared to have the formulations 1 to 4 as shown in Table 1 below. Formulation 4 is according to the invention. Formulations 1-3 are comparative.

Pure water (reverse osmosis-grade) was used to prepare all formulations. The water was then acidified using sulphuric acid to a pH value of 3.5. Sterile stock solutions of all mineral additions (with the exception of calcium sulphate) and of other additives were prepared and added in appropriate amounts to achieve the concentrations set out in Table 1. Since calcium sulphate is insoluble in water, formulations containing Ca²⁺ ions were prepared by adding the appropriate amount of pure solid CaSO₄2H₂O to the acidified mineral solution and allowing this to dissolve. After the preparation of each iron solution formulation, the pH was adjusted to a value of 3.0 using sulphuric acid and then each solution was filter sterilised (through 0.2 µm pore-size membrane filters) into a heat-sterilised bottle and stored.

**TABLE 1**

| Component | Concentration (mg/l) | | | |
|---|---|---|---|---|
| | 1* | 2* | 3* | 4 |
| Ferrous Iron | 200 | 200 | 200 | 200 |
| Ferric Iron | 20 | 20 | 20 | 20 |
| Calcium | 170 | 0 | 170 | 0 |
| Magnesium | 0 | 40 | 40 | 0 |
| Sodium | 0 | 0 | 0 | 0 |
| Manganese | 0 | 0 | 0 | 0 |
| Potassium | 0 | 0 | 0 | 0 |
| Zinc | 0 | 0 | 0 | 40 |
| Apple Concentrate | 0 | 0 | 0 | 0 |
| Ascorbic Acid | 0 | 0 | 0 | 0 |
| Potassium Sorbate | 0 | 0 | 0 | 0 |
| | | | | |
| pH | 3.0 | 3.0 | 3.0 | 3.0 |

| | | | | |
|---|---|---|---|---|
| * Comparative | | | | |

### Iron uptake assay

### Summary

Iron uptake was determined by measuring the amount of intracellular ferritin produced by human intestinal epithelial Caco-2 cells incubated with the sample to be assayed. After incubation, the cells were harvested and the amount of ferritin measured using an enzyme-linked immunosorbent assay (ELISA) technique involving spectrophotometric testing of the resulting solution. The concentration of ferritin is directly proportional to the colour intensity of the solution.

### Caco-2 iron uptake - Experimental Protocol

1. Caco-2 cells were seeded (50,000/cm2) into 6-well cell culture plates (2ml media/well). The media was replenished every 48 hours.
2. The cells were differentiated fully by day 14 post-seeding. Therefore, the experiments were carried out between days 14 - 18 post-seeding. Day 13 post-seeding growth media was removed and the cells were washed twice with wash solution (Hank's buffered salt solution (HBSS) at 37°C or using 140mM NaCl, 5mM KCl and 10mM piperazine-N,N'-bis(2-ethanesulphonic acid) (PIPES) buffer, pH 6.7, 37°C) and incubated in serum-free Minimum Essential Media (MEM) for 24 hours.
3. Test media were prepared in iron-free controlled media (serum-free MEM). Test media pH was adjusted to 5.8 (using 1M HCl and 1M NaOH). Iron formulations for testing were prepared by subjecting each to simulated dissolution conditions of stomach (0.1M HCl, pH 1.8, 37°C, 2-6 hours with magnetic stirring). An aliquot of the sample for testing (equivalent to 20µm elemental iron) was withdrawn for addition to the Caco-2 cells.
4. Media aspirated (cells washed with wash solution) and test media for each condition were added in triplicate to a 6-well plate. Volume from test and control samples were added to test media at a final concentration of 20µm elemental iron.
5. The cell-containing mixture was incubated for 2-24 hours at 37°C on a rotary shaker (6 RPM). After the end of the incubation period, the media was aspirated and cells were washed twice with wash solution Growth medium (serum-free MEM, pH 5.8) was added to the cells and the cells were then incubated overnight (24 hours total incubation time).
6. Cell harvesting: media was aspirated, cells were washed with wash solution and then with removal solution (wash solution + 5µm Na hydrosulphite and 1µm bathophenanthroline, sulphonated sodium salt (4,7-diphenyl-1,10-phenanthrolinedisulphnic acid disodium salt (BPDS)) to remove surface-bound iron. The cells were then washed again with wash solution.
7. The cells, harvested according to 6. above, were treated by adding 350µl lysis buffer to each well and the plates were incubated on a plate rocker (8 RPM) for 40 minutes (in ice trays).
8. The cell lysate was collected and pipetted into a microcentrifuge tube (Eppendorf).
9. The cell lysate was passed at least five times through a 1ml syringe fitted with a 25G needle.
10. The microcentrifuge tube containing the cell lysate was centrifuged at 13,000 rpm for 10 minutes at 4°C. The supernatant was collected and stored at -20°C.

The iron formulations identified in Table 1 above were each treated and incubated with Caco-2 cells in accordance with the above protocol. In addition, a control solution containing 200 mg/l of Fe²⁺ and 20 mg/l of Fe³⁺, pH 3.0 was also treated and incubated in the same way as the iron formulations in Table 1. The cell lysate obtained in each case was subjected to ferritin ELISA to quantitatively determine the amount of ferritin.

The quantitative analysis of ferritin was determined using a 'Spectro Ferritin' kit (Ramco Laboratories, Inc., US) according to the experimental procedure set out below.

### Ferritin ELISA Procedure

1. The samples for testing were centrifuged at 13000 rpm for 10 minutes at 4°C.
2. 30µl of each standard, each blank and each sample for testing were pipetted into duplicate microwells coated with rabbit antihuman spleen ferritin. No additions were made to microwells used to measure nonspecific binding (NSB).
3. 200µl alkaline phosphatase conjugated rabbit antihuman spleen ferritin were added to all microwells.
4. The microwells with contents were incubated at room temperature for 2 hours on a rotator table set at 195 rpm.
5. The microwells were washed with deionised water by filling each microwell with water and then shaking to decant. This washing procedure was repeated 3 times. After the final washing, the tops of the microwells were tapped on absorbent material for about 30 seconds to drain.
6. 200µm of substrate solution (phenylphosphate disodium and 4-aminoantipyrine in 10% diethanolamine) was pipetted into each microwell.
7. The microwells containing substrate solution were then incubated for 30 minutes at room temperature.
8. 100µl of 0.24% potassium ferricyanide were added to each microwell to cause colour development and the contents of the microwells were mixed thoroughly.
9. Absorbances were measured spectrophotometrically at 490nm and at a correction wavelength of 630nm. The optical density is directly proportional to the ferritin concentration in the sample. Ferritin concentrations were calculated as per kit manufacturer's instructions.

The iron uptake by Caco-2 cells (as ferritin) obtained for each of the iron formulations identified in Table 1 above (average of three values) are set out in Table 2 below. The table also shows iron uptake by Caco-2 cells (as %) normalised against the Fe²⁺/Fe³⁺ control described above (taken as 100%).

**TABLE 2**

| Iron formulation | 1* | 2* | 3* | 4 |
|---|---|---|---|---|
| Iron uptake as ferritin (ng/mg protein) | 164 | 218 | 268 | 285 |
| % based on Fe²⁺/Fe³⁺ control | 103 | 138 | 170 | 180 |

| | | | | |
|---|---|---|---|---|
| * Comparative | | | | |

### Example 5

A mineral water composition according to the invention was prepared to have the formulation : 220 mg/l ferrous ion, 40 mg/l zinc ion, 1320 mg/l potassium sorbate, with pH adjusted using sulphuric acid. Iron bioavailability by comparison of ferritin formation was assessed. The composition showed a high level of bioavailable iron.

## Claims

1. A non-toxic artificial mineral water composition containing bioavailable iron, said composition consisting of an aqueous solution having a pH of from 1 to 5 containing ferrous ions at a concentration of from 100 to 300 mg/l and a non-toxic amount of Zn²⁺ ions wherein the concentration of Zn²⁺ ions is in the range of from 20 to 60 mg/l, and wherein the composition, when diluted to an iron concentration of 20 µm, has an iron uptake, measured as intracellular ferritin, of greater than 120 ng/mg protein, wherein iron uptake is determined by incubating human intestinal epithelial Caco-2 cells according to the method provided in the examples.

2. A mineral water composition according to claim 1, wherein the concentration of Zn²⁺ ions is in the range of from 20 to 50 mg/l.

3. A mineral water composition according to either claim 1 or claim 2, wherein the concentration of ferrous ions is in the range of from 150 to 250 mg/l.

4. A mineral water composition according to any one of claims 1 to 3, further containing ferric ions at a concentration not greater than 70 mg/l, preferably not greater than 50 mg/l.

5. A mineral water composition according to any one of claims 1 to 4, wherein potassium is present in the form of potassium sorbate.

6. A mineral water composition according to claim 5, wherein the concentration of potassium sorbate is not greater than 1800 mg/l, and is preferably in the range of from 1000 to 1500 mg/l.

7. A mineral water composition according to any one of claims 1 to 6 which additionally contains one or more flavour components.

8. A mineral water composition according to claim 7, wherein the flavour component is selected from apple juice, citrus juice and Vitamin C.

9. A mineral water composition according to any one of claims 1 to 8, which has a pH in the range of from 2 to 5, preferably in the range of from 2 to 4 and more preferably in the range of from 2.5 to 3.5.

## Patentansprüche

1. Nichttoxische künstliche Mineralwasserzusammensetzung, die bioverfügbares Eisen enthält, wobei die Zusammensetzung aus einer wässrigen Lösung mit einem pH-Wert von 1 bis 5 besteht, die Eisenionen in einer Konzentration von 100 bis 300 mg/l und eine nichttoxische Menge von Zn²⁺-Ionen enthält, wobei die Konzentration von Zn²⁺-Ionen im Bereich von 20 bis 60 mg/l liegt und wobei die Zusammensetzung bei Verdünnung auf eine Eisenkonzentration von 20 µm eine Eisenaufnahme, gemessen als intrazelluläres Ferritin, von über 120 ng/mg Protein aufweist, wobei die Eisenaufnahme durch Inkubieren von Caco-2-Darmepithelzellen gemäß dem in den Beispielen bereitgestellten Verfahren bestimmt wird.

2. Mineralwasserzusammensetzung nach Anspruch 1, wobei die Konzentration von Zn²⁺-Ionen im Bereich von 20 bis 50 mg/l liegt.

3. Mineralwasserzusammensetzung nach Anspruch 1 oder 2, wobei die Konzentration von Eisenionen im Bereich von 150 bis 250 mg/l liegt.

4. Mineralwasserzusammensetzung nach einem der Ansprüche 1 bis 3, ferner enthaltend Eisenionen in einer Konzentration von höchstens 70 mg/l, bevorzugt höchstens 50 mg/l.

5. Mineralwasserzusammensetzung nach einem der Ansprüche 1 bis 4, wobei Kalium in Form von Kaliumsorbat vorhanden ist.

6. Mineralwasserzusammensetzung nach Anspruch 5, wobei die Konzentration von Kaliumsorbat höchstens 1 800 mg/l beträgt und bevorzugt im Bereich von 1 000 bis 1 500 mg/l liegt.

7. Mineralwasserzusammensetzung nach einem der Ansprüche 1 bis 6, die außerdem eine oder mehrere Geschmacksstoffkomponenten enthält.

8. Mineralwasserzusammensetzung nach einem der Ansprüche 1 bis 7, die Geschmacksstoffkomponente aus Apfelsaft, Zitrussaft und Vitamin C ausgewählt ist.

9. Mineralwasserzusammensetzung nach einem der Ansprüche 1 bis 8, die einen pH-Wert im Bereich von 2 bis 5, bevorzugt im Bereich von 2 bis 4 und besonders bevorzugt im Bereich von 2,5 bis 3,5 aufweist.

## Revendications

1. Composition d'eau minérale artificielle non toxique contenant du fer biodisponible, ladite composition étant constituée d'une solution aqueuse ayant un pH de 1 à 5 contenant des ions ferreux à une concentration de 100 à 300 mg/l et une quantité non toxique d'ions Zn²⁺ la concentration d'ions Zn²⁺ étant située dans la plage de 20 à 60 mg/l, et la composition, quand elle est diluée à une concentration en fer de 20 µm, ayant une absorption de fer, mesurée comme ferritine intracellulaire, de plus de 120 ng/mg de protéine, l'absorption de fer étant déterminée par l'incubation de cellules Caco-2 épithéliales intestinales humaines selon le procédé fourni dans les exemples.

2. Composition d'eau minérale selon la revendication 1, avec laquelle la concentration d'ions Zn²⁺ se situe dans la plage de 20 à 50 mg/l.

3. Composition d'eau minérale selon la revendication 1 ou la revendication 2, avec laquelle la concentration d'ions ferreux se situe dans la plage de 150 à 250mg/l.

4. Composition d'eau minérale selon l'une quelconque des revendications 1 à 3, contenant en outre des ions ferriques à une concentration de pas plus de 70 mg/l, de préférence de pas plus de 50 mg/l.

5. Composition d'eau minérale selon l'une quelconque des revendications 1 à 4, avec laquelle du potassium est présent sous forme de sorbate de potassium.

6. Composition d'eau minérale selon la revendication 5, avec laquelle la concentration de sorbate de potassium est de pas plus de 1800 mg/l, et se situe de préférence dans la plage de 1000 à 1500 mg/l.

7. Composition d'eau minérale selon l'une quelconque des revendications 1 à 6 qui contient de plus un ou plusieurs composants de saveur.

8. Composition d'eau minérale selon la revendication 7, avec laquelle le composant de saveur est choisi parmi le jus de pomme, le jus de citron et la vitamine C.

9. Composition d'eau minérale selon l'une quelconque des revendications 1 à 8, qui a un pH dans la plage de 2 à 5, de préférence dans la plage de 2 à 4 et davantage de préférence dans la plage de 2,5 à 3,5.
